(19) Europäisches Patentamt
European Patent Office
Office européen des brevets

(11) Numéro de publication: **0 077 281**
**B1**

# (12) FASCICULE DE BREVET EUROPEEN

(45) Date de publication du fascicule du brevet: **13.02.85**

(51) Int. Cl.⁴: **C 07 C 153/09**, C 07 D 307/68, A 01 N 37/46, A 01 N 43/08

(21) Numéro de dépôt: **82420142.0**

(22) Date de dépôt: **04.10.82**

## (54) Nouveaux fongicides homologués des N-phényl alaninates et leur procédé de préparation et leur application.

(30) Priorité: **06.10.81 FR 8118915**

(43) Date de publication de la demande: **20.04.83 Bulletin 83/16**

(45) Mention de la délivrance du brevet: **13.02.85 Bulletin 85/7**

(84) Etats contractants désignés: **BE CH DE FR GB IT LI LU NL**

(56) Documents cités:
**EP - A - 0 045 049**
**FR - A - 2 176 075**
**FR - A - 2 267 042**
**FR - A - 2 326 416**

**Le dossier contient des informations techniques présentées postérieurement au dépôt de la demande et ne figurant pas dans le présent fascicule.**

(73) Titulaire: **RHONE-POULENC AGROCHIMIE, 14-20, rue Pierre Baizet, F-69009 Lyon (FR)**

(72) Inventeur: **Lacroix, Guy, 332 C Balmont La Duchère, F-69009 Lyon (FR)**
Inventeur: **Debourge, Jean-Claude, 14, Avenue des Frères Lumière, F-69410 Champagne au Mont d'Or (FR)**

(74) Mandataire: **Brachotte, Charles et al, RHONE-POULENC AGROCHIMIE P.I.D. BP 9163, F-69263 Lyon Cedex 09 (FR)**

**EP 0 077 281 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne de nouveaux produits à groupement amide dérivant de l'aniline. L'invention concerne aussi la préparation desdits produits et leur application pour la protection des végétaux, spécialement contre les champignons et les maladies fongiques.

On connait divers produits antifongiques de la famille des N-phénylalaninates qui sont décrits notamment dans les brevets français 2 267 042 et 2 326 416. L'un de ces produits a même donné lieu à une commercialisation.

Un but de l'invention est de fournir des produits ayant une activité remarquablement améliorée par rapport aux produits connus, notamment vis à vis du mildiou de la tomate et du pythium.

Les produits composés selon l'invention sont caractérisés en ce qu'ils ont pour formule

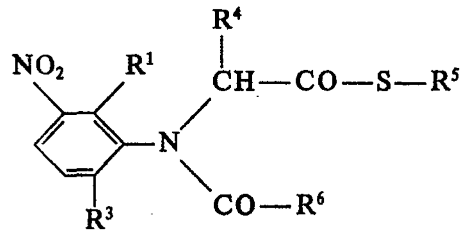

(I)

dans laquelle:

- $R^1$ et $R^3$ identiques ou différents, représentent un radical alkyle ayant de 1 à 4 atomes de carbone et sont de préférence le radical méthyle,
- $R^4$ représente l'atome d'hydrogène ou un radical méthyle,
- $R^5$ représente l'atome d'hydrogène ou un radical alkyle ayant de 1 à 4 atomes de carbone,
- $R^6$ représente un radical alkyle, éventuellement mono- ou dihalogéné, ayant de 1 à 6 atomes de carbone, ou un radical cycloalkyle ayant de 3 à 6 atomes de carbone ou un radical $-CH_2-X-R^7$, ou un hétérocycle furylique,
- X représente l'atome d'oxygène ou de soufre,
- $R^7$ représente un radical alkyle ayant de 1 à 4 atomes de carbone.

Dans la famille générale de composés de formule (I) telle que l'on vient de la définir, une sous-famille particulière est spécialement avantageuse; elle présente notamment de très bonnes propriétés antifongiques spécialement à l'égard des phycomycètes, et plus particulièrement à l'égard des mildious; ces produits préférés sont tels que $R^1$, $R^3$, $R^4$ et, le cas échéant, $R^7$ représentent un groupe méthyle.

Dans ces composés préférés la présence d'un carbone asymétrique (isomérie optique) sur la chaine

$$-CH(R^4)-CO-S-R^5$$

d'une part, et, d'autre part la dissymétrie (également appelée atropoisomérie) créée par le substituant $NO_2$ sur le noyau phényle, provoquent l'apparition de deux stéréoisomères (ou pseudo-diastéréoisomères) observables par divers moyens analytiques et isolables par des moyens physiques. Ces stéréoisomères font également partie de l'invention.

Les composés selon l'invention peuvent être préparés de diverses manières.

Selon un premier procédé on procède à une acylation d'un composé de formule (II) avec un chlorure d'acide de formule $R^6-CO-Cl$ (III) selon le schéma:

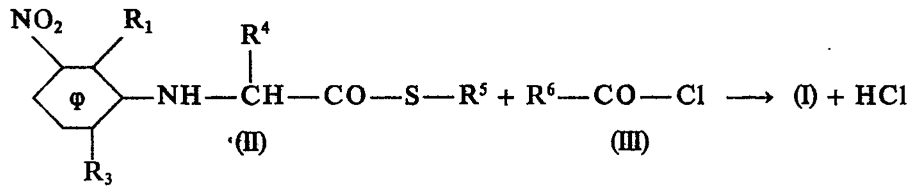

La réaction mise en œuvre dans ce procédé s'effectue avantageusement en solution dans un solvant inerte (c'est-à-dire ne réagissant pas chimiquement avec les réactifs dans les conditions opératoires). La température de réaction est généralement supérieure à 40°C et inférieure à la température de dégradation des réactifs et/ou produits de réaction. La température est ainsi généralement comprise entre 60 et 150°C. Selon une modalité commode on opère à la température d'ébullition du solvant considéré. Comme solvant approprié on peut citer les solvants organiques aprotiques polaires ou non polaires. On préfère utiliser les solvants dont le point d'ébullition est compris dans la zone indiquée pour la température de réaction; il peut donc s'agir d'hydrocarbures aromatiques (tels que le benzène,

le toluène, les xylènes, le chlorobenzène) ou aliphatiques (tels que l'hexane, l'heptane, le cyclohexane, le méthylcyclohexane) ou d'hydrocarbures aliphatiques chlorés (tels que le dichloroéthane, le dichloroéthylène, le chloroforme, le tétrachlorure de carbone) ou d'éthers (tels que le dioxanne, le tétrahydrofurane, l'éther diéthylique) ou de cétones (telles que l'acétone, la méthyléthylcétone, la méthylisobutylcétone) ou de nitriles (tels que l'acétonitrile).

La réaction peut être catalysée, par exemple par du diméthylformamide; elle peut être effectuée en absence ou en présence d'agent de condensation, notamment d'accepteur d'acide. Comme accepteurs d'acide on peut utiliser des amines tertiaires telles que les trialkylamines (par exemple la triéthylamine) ou les N-aryldialkylamines (par exemple la N,N-diméthylaniline) ou encore la pyridine et les bases pyridiniques ou les bases minérales telles que les carbonates et hydrogénocarbonates de métaux alcalin ou alcalinoterreux, et l'acétate de sodium.

Il est possible déffectuer la réaction en présence d'un excès de l'un ou l'autre des réactifs. Une modalité préférée consiste cependant à opérer en présence d'un excès de composé de formule (II) par exemple en introduisant progressivement le chlorure d'acide de formule (III) dans le milieu réactionnel contenant tout ou partie du composé de formule (II) devant être mis en œuvre, puis l'on poursuit la réaction jusqu'à cessation du dégagement d'acide chlorhydrique; les quantités de réactifs mis en œuvre globalement au cours de la réaction sont de préférence voisines de la stoechiométrie; la stoechiométrie est généralement la plus avantageuse; toutefois le chlorure d'acide de formule (III) peut être mis en œuvre en quantité dépassant la stoechiométrie, par exemple jusqu'à 10% (en nombre). En fin de réaction on isole le produit de réaction de formule (I) par tout moyen connu en soi, par exemple par distillation du solvant (c'est-à-dire évaporation) et/ou cristallisation du produit dans le milieu.

En ce qui concerne la préparation des composés de formule (II), elle peut s'effectuer selon des procédés semblables à ceux décrits pour la préparation d'esters anilino alcanecarboxyliques dans les publications suivantes: J. Org. Chem. 30 p. 4101—4104 (1965), Tetrahedron 1967 p. 487—498.

La préparation des chlorures d'acides de formule (III) peut être faite conformément ou semblablement à bien des procédés connus mais spécialement ceux décrits dans

— le brevet des Etats-Unis d'Amérique n° 2 412 700,
— Berichte 46 p. 2103—2107 (1913),
— Annalen 602 p. 1—14 (1957).

Les composés de l'invention peuvent être préparés selon un second procédé, par estérification d'un acide de formule (IV) avec un mercaptan de formule $R^5SH$ selon le schéma:

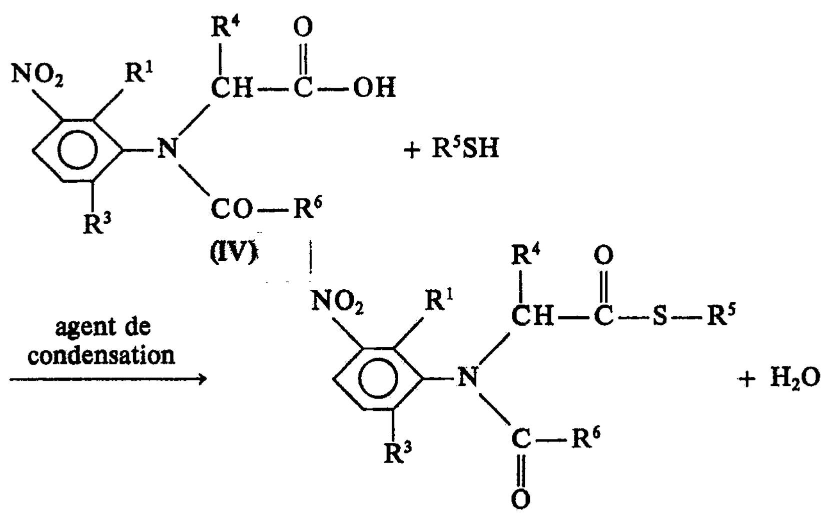


La réaction d'estérification peut être réalisée par toutes les techniques connues en soi.

On peut notamment utiliser comme agent de déshydratation les carbodimides et plus particulièrement le dicyclohexylcarbodimide.

Dans ce cas la réaction s'effectue le plus commodément à des températures comprises entre 10 et 120° C, dans un solvant organique inerte. Comme solvant utilisable on peut citer les solvants utilisables dans le premier procédé.

Comme agent de déshydratation on peut citer les carbodiimides, notamment le dicyclohexylcarbodiimide. Les quantités d'agent de déshydratation et d'acide (IV) sont de préference voisines de la stoechiométrie (au moins 90% de la stoechiométrie par rapport au mercaptan de formule $R^5SH$).

Sur un plan pratique on préfère aussi ajouter progressivement le carbodiimide à un mélange de mercaptan et d'acide (IV).

Au cours de la réaction il se forme généralement une urée substituée qui s'élimine en fin de réaction

par tout moyen connu en soi, par exemple par filtration lorsque cette urée substituée est insoluble. Le solvant est également éliminé, par exemple par évaporation, ce qui conduit à l'isolement du composé de formule (I).

Les acides de formule (IV) peuvent être préparés par saponification des composés de formule (V)

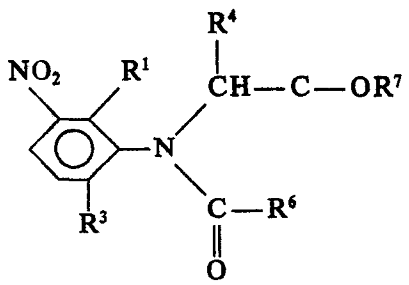

Ces composés de formule (V) peuvent être préparés selon les procédés décrits dans le brevet français n° 2 267 042.

Les exemples suivants donnés à titre non limitatif, illustrent l'invention et montrent comment elle peut être mise en pratique.

L'exemple 1 illustre la préparation d'un composé selon le premier procédé.

L'exemple 2 illustre la préparation d'un composé selon le second procédé.

L'exemple 3 illustre la séparation de pseudio-diastéréoisomères.

Les autres exemples illustrent l'application des produits des exemples 1 à 3 et du tableau (I).

Les pulvérisations de solutions ou suspensions de matières actives décrites dans les exemples 4 et 5 sont effectuées dans des conditions telles que la pulvérisation d'une solution ou suspension de concentration égale à 1 g/l correspond en moyenne à l'application d'environ 2 microgrammes de matière active par $cm^2$ de feuille de la plante.

Dans les conditions des exemples 4 et 5, les composés illustrés n'ont pas présenté de phytotoxicité.

Dans ces exemples 4 et 5 on considère qu'un produit exerce une protection totale vis-à-vis d'une maladie fongique lorsque la protection est d'au moins 95%; la protection est considérée comme bonne lorsqu'elle est d'au moins 80% (mais inférieure à 95%), comme assez bonne lorsqu'elle est d'au moins 70% (mais inférieure à 80%), comme moyenne lorsqu'elle est d'au moins 50% (mais inférieure à 70%).

## Exemple 1

### Préparation de N-(diméthyl-2,6 nitro-3 phényl) N-chloracétyl thiolalanisate de méthyle (composé n° 6)

Dans 50 ml de toluène, on dissout 5,4 g (0,02 mole) de N-(diméthyl-2,6 nitro-3 phényl) thiolalaninate de méthyle et 2,3 g de chlorure de chloracétyle. On chauffe progressivement le milieu réactionnel jusqu'à l'ébullition à reflux: on observe alors un dégagement d'acide chlorhydrique. Le chauffage à reflux est maintenu jusqu'à cessation du dégagement d'acide chlorhydrique, c'est-à-dire durant 3 heures. Le milieu réactionnel est refroidi à température ambiante puis lavé 3 fois avec 50 ml d'eau chaque fois. La solution finale est séchée puis concentrée. L'huile résiduelle est cristallisée à l'aide d'un mélange hexane/éther. Les cristaux sont dispersés dans le solvant puis filtrés et séchés sous vide.

On obtient ainsi 6 g d'un solide beige fondant à 113° C.

## Exemple 2

### Préparation de N-(diméthyl-2,6 nitro-3 phényl) N-méthoxyacétyl thiolglycinate de méthyle (composé n° 11)

Dans 80 $cm^3$ d'acétonitrile on dissout:

– 5,9 g de N-(diméthyl-2,6 nitro-3 phényl)-N-méthoxyacétyl glycine de formule:

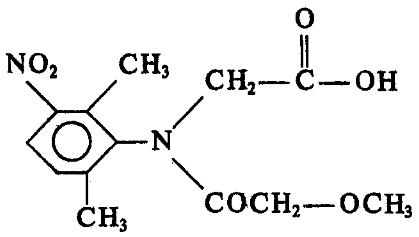

— 1,1 g de méthylmercaptan ($CH_3SH$).

On agite à 20° C et ajoute rapidement une solution de 4,5 g de dicyclohexylcarbodiimide dans 20 $cm^3$ d'acétonitrile.

On chauffe le milieu réactionnel dans un autoclave pendant 2 h à 80° C puis on le refroidit à température ambiante. On filtre le précépité de dicyclohexylurée, élimine le solvant par distillation, ajoute 200 ml de chlorure de méthylène. On lave la solution chlorométhylénique avec une solution de bicarbonate de sodium puis à l'aide d'eau. Après distillation du solvant on reprend le résidu avec 20 ml d'éther: un solide beige cristallisé. Le produit est filtré puis séché sous vide.

On obtient ainsi 4,5 g d'une poudre beige clair.

Après purification par chromatographie sur colonne de silice on obtient un produit qui fond à 102° C.

Exemple 3

Séparation des pseudo-diastéréoisomères du N-(diméthyl-2,6 nitro-3 phényl)-N-méth oxyacétyl thiolalaninate de méthyle (composés n° 1 et 2).

La synthèse de ce dernier composé cité est effectuée selon le premier procédé décrit; on obtient un mélange équimolaire des deux pseudodiastéréoisomères de ce composé.

La chromatographie sur couche mince de silice (plaque Merck: Kieselgel 60F-254) utilisant comme système éluant un mélange toluène/acétate d'éthyle en proportions volumiques respectives (70—30) permet de distinguer les deux stéréoisomères, l'un ayant un Rf égal à 0,36, l'autre ayant un Rf égal à 0,25. Par Rf on désigne le rapport des hauteurs finales de migration du composé considéré par rapport au système éluant. Ces deux stéréoisomères, sont également distingués en spectrographie RMN qui indique que les proportions de ces stéréoisomères sont pratiquement égales. Par convention on apelle isomère A celui ayant le Rf le plus élevé dans le système décrit ci-dessus et isomère B celui ayant le Rf le plus faible.

$$Rf(A) > Rf(B)$$

Les deux isomères sont séparés par chromatographie sur colonne de silice en utilisant le même système que celui décrit ci-dessus. Chacun des isomères cristallise dans l'éther diéthylique.

L'isomère A se présente sous forme d'un solide jaune pâle fondant à 95,7° C.

L'isomère B se présente également sous forme d'un solide jaune pâle fondant à 139° C.

D'une manière semblable à celle décrite dans ces exemples 1 à 3 on prépare les composés n° 1 à 11 dont les caractéristiques sont indiquées dans le tableau (I) et qui ont pour formule générale:

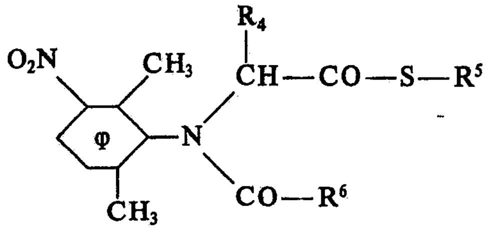

Exemple 4

Utilisation in vivo sur Plasmopara viticola sur plants de vigne (traitement préventif)

Des plants de vigne (cépage GAMAY), cultivés en pots sont traités sur les deux faces de leurs feuilles par pulvérisation d'une émulsion aqueuse contenant la matière active à tester; l'émulsion pulvérisée est constituée de:

- 40 mg de matière active à tester
- 40 $cm^3$ d'eau
- 0,02 $cm^3$ de Tween 80 (agent tensioactif constitué d'un oléate de dérivé polyoxyéthyléné du sorbitol).

Cette émulsion, ainsi constituée, permet la pulvérisation d'une émulsion aqueuse contenant 1 g/l de matière active à tester. Pour obtenir des émulsions à pulvériser de concentrations en matière active à tester inférieures à 1 g/l, on dilue à l'eau l'émulsion aqueuse ainsi constituée.

Au bout de 48 heures, la contamination est effectuée par pulvérisation, sur la face inférieure des feuilles, d'une suspension aqueuse de 80 000 unités/$cm^3$ environ de spores du champignon. Ensuite, les pots sont placés pendant 48 heures en cellule d'incubation à 100% d'humidité relative et à 20° C.

On effectue le contrôle des plants 9 jours après l'infestation.

Tableau (I)

| Compose No. | $R_4$ | $R_5$ | $R_6$ | Isomere | Point de Fusion |
|---|---|---|---|---|---|
| 1 | $CH_3$— | $CH_3$— | $CH_3OCH_2$ | Isomère A | 95°C |
| 2 | $CH_3$— | $CH_3$— | $CH_3OCH_2$— | Isomère B | 139°C |
| 3 | $CH_3$— | $CH_3$— | $CH_3SCH_2$— | Isomère A | 105°C |
| 4 | $CH_3$— | $CH_3$— | $CH_3SCH_2$— | Isomère B | 117°C |
| 5 | $CH_3$— | $CH_3$— | $CH_3$— | Mélange isomères | 108°C |
| 6 | $CH_3$— | $CH_3$— | Cl $CH_2$— | Mélange isomères | 113°C |
| 7 | $CH_3$— | $CH_3$— | Cyclopropyl | Isomère A | 70°C |
| 8 | $CH_3$— | $CH_3$— | Cyclopropyl | Isomère B | 153°C |
| 9 | $CH_3$— | $CH_3$— | 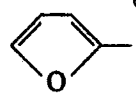 | Isomère A | 86°C |
| 10 | $CH_3$— | $CH_3$— | 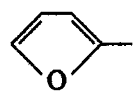 | Isomère B | 151°C |
| 11 | H | $CH_3$— | $CH_3OCH_2$— | | 102°C |

Dans ces conditions d'utilisation, on observe: A la dose de 10 mg/l, une protection totale est assurée pour les composés 6, 7, 8, 9, 10 et 11.

A la dose de 33 mg/l, on observe une protection totale pour les composés 6, 7 et 9, une bonne protection pour le composé 10.

A la dose de 11 mg/l, les composés 7 et 9 assurent encore une protection totale et les composés 6 et 10 assurent une bonne protection.

## Exemple 5

### Utilisation in vivo sur »Phytophthora Infestans« responsable du mildiou de la tomate

Des plants de tomates (variété Marmande) cultivés en serre et agés de 60 à 75 jours sont traités par pulvérisation avec des émulsions aqueuses préparées comme indiqué à l'exemple 4 et contenant diverses concentrations de matière active à tester.

Au bout de 48 heures, les plants traités sont contaminés avec une suspension aqueuse de spores (zoosporanges) obtenue à partir d'une culture de »Phytophthora Infestans« cultivée pendant 20 jours

sur un milieu à base de farine de pois chiches.

Les plants de tomate sont placés pendant 48 heures dans une enceinte à une température de 16 à 18°C et munie d'une atmosphère ayant une humidité relative de 100% puis l'humidité relative est ensuite ramenée à 80%.

On observe les résultats 8 jours après contamination. Les résultats s'apprécient par évaluation de la surface de feuilles envahie par le champignon et s'expriment par le »pourcentage de protection« c'est-à-dire $100\left(1 \frac{S}{Stm}\right)$, S étant la surface envahie par le champignon sur le plant considéré et Stm étant la surface envahie par le champignon sur le plant témoin non traité. On indique ci-après les résultats, comme dans les exemples précédents, sous forme de: protection totale, bonne, assez bonne ou moyenne.

Dans ces conditions et avec une émulsion aqueuse de concentration 33 mg/l en matière active à tester, on a observé une protection totale avec les composés n° 1 et 2 et une bonne protection avec le composé n° 3.

A la concentration de 11 mg/l de matière active on observe une protection totale avec le composé de l'exemple 1 et une bonne protection avec le composé de l'exemple 2.

A la concentration de 3 mg/l en matière active, on observe une protection totale avec le composé n° 1.

## Exemple 6

### Essais in vitro sur Pythium sp

Le composé à tester est ajouté sous forme de solution acétonique (à 1%) dans un tube à essai contenant un milieu de culture stérile et en surfusion (60°C). Après mélange, le milieu contenant le produit est coulé aseptiquement dans une boite de Petri (diamètre: 5 cm). On réalise ainsi des séries de boites contenant des doses de matière active respectivement égales à 0,3 — 1 — 3 — 10 — 30 mg/l. Après 24 heures les boites sont inoculées par dépôt au centre d'un implant de mycelium (diamètre 8 mm) du champignon étudié.

On compare ensuite la vitesse de croissance du champignon sur milieu sans produit (témoin) et sur milieu contenant les doses préalablement décrites; l'appréciation de la vitesse de croissance des champignons se fait par mesure du diamètre de la colonie.

La notation des résultats s'effectue selon le barème suivant:

95 à 100% d'inhibition de la croissance — note 4
75 à 95% d'inhibition de la croissance — note 3
50 à 75% d'inhibition de la croissance — note 2
25 à 50% d'inhibition de la croissance — note 1
moins de 25% d'inhibition de la croissance — note 0.

Les résultats sont rassemblés dans le tableau (II).

Tableau (II)

| Dose d'application en mg/l | No. du composé utilisé 1 | 2 | 6 | 7 | 9 | 10 |
|---|---|---|---|---|---|---|
| 10 | 4 | 3 | | | | |
| 3 | 4 | 2 | 4 | 4 | 4 | 3 |
| 1 | 4 | 1 | 3 | 3 | 4 | 2 |

Ces expérimentations illustrent clairement les remarquables propriétés fongicides des composés selon l'invention, spécialement sur les champignons de type Phycomycètes, ainsi que leur absence de phytotoxicité.

Pour leur emploi pratique, les composés selon l'invention sont rarement utilisés seuls. Le plus souvent ils font partie de compositions. Ces compositions, utilisables pour la protection des végétaux contre les maladies fongiques, contiennent comme matière active un composé selon l'invention tel que décrit précédemment en association avec les supports solides ou liquides, acceptables en agriculture et les agents tensio-actifs également acceptables en agriculture. En particulier sont utilisables les supports inertes et usuels et les agents tensio-actifs usuels.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc. ... ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Les doses d'emploi des composés selon l'invention peuvent varier dans de larges limites, notamment selon la virulence des champignons et les conditions climatiques.

D'une manière générale des compositions contenant 0,5 à 5000 ppm de substance active conviennent bien; ces valeurs sont indiquées pour les compositions prêtes à l'application. Ppm signifie »partie par million«. La zone de 0,5 à 5000 ppm correspond à une zone de $5 \cdot 10^{-5}$ à 0,5% (pourcentages pondéraux).

En ce qui concerne les compositions adaptées au stockage et au transport, elles contiennent plus avantageusement de 4 à 95% (en poids) de substance active.

Ainsi donc, les compositions à usage agricole selon l'invention peuvent contenir les matières actives selon l'invention dans de très larges limites, allant de $5 \cdot 10^{-5}$% à 95% (en poids).

Selon ce qui a déjà été dit les composés selon l'invention sont généralement associés à des supports et éventuellement des agents tensioactifs.

Par le terme »support«, dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc. . .) ou liquide (eau, alcools, cétones, fractions de pétrole, hydrocarbures aromatiques ou paraffiniques, hydrocarbures chlorés, gaz liquéfiés, etc. . .).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100%) et les granulés, notamment ceux obtenus par extrusion, par compactage, par imprégnation d'un support granulé, par granulation à partir d'une poudre (la teneur en composé de formule (I) dans ces granulés étant entre 1 et 80% pour ces derniers cas).

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80% de matière active, les émulsions ou solutions prêtes à l'application contenant, quant à elles, 0,001 à 20% de matière active. En plus du solvant, les concentrés émulsionnables peuvent contenir, quand c'est nécessaire, 2 à 20% d'additifs appropriés, comme des stabilisants, des agents tensioactifs, des agents de pénétration, des inhibiteurs de corrosion, des colorants, des adhésifs. A titre d'exemple, voici la composition de quelques concentrés:

| | |
|---|---|
| — matière active | 400 g/l |
| — dodécylbenzène sulfonate alcalin | 24 g/l |
| — nonylphénol oxyéthylé à 10 moléoules d'oxyde d'éthylène | 16 g/l |
| — cyclohexanone | 200 g/l |
| — solvant aromatique | g.s.p 1 litre |

Selon une autre formule de concentré émulsionnable, on utilise:

| | |
|---|---|
| — matière active | 250 g |
| — huile végétale époxydée | 25 g |
| — mélange su sulfonate d'alcoylaryle et d'éther de polyglycol et d'alcools gras | 100 g |
| — diméthylformamide | 50 g |
| — xylène | 575 g |

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75% de matière active, de 0,5 à 15% d'agents tensioactifs, de 0,1 à 10% d'agents thixotropes, de 0 à 10% d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble: certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95% de matière active, et elles contiennent habituellement, en plus du support solide, de 0 à 5% d'un agent mouillant, de 3 à 10% d'un agent dispersant, et, quand c'est nécessaire, de 0 à 10% d'un ou plusieurs stabilisants et/ou autres additifs, comme des agents de pénétration, des adhésifs, ou des agents antimottants, colorants, etc...

A titre d'exemple, voici diverses compositions de poudres mouillables:

| | |
|---|---|
| — matière active | 50% |
| — lignosulfonate de calcium (défloculant) | 5% |
| — isopropylnaphtalène sulfonate (agent mouillant anionique) | 1% |
| — silice antimottante | 5% |
| — kaolin (charge) | 39% |

Une autre composition de poudre à pulvériser à 70% utilise les constituants suivants:

| | |
|---|---|
| — matière active | 700 g |
| — dibutylnaphtylsulfonate de sodium | 50 g |
| — produit de condensation en proportions 3/2/1 d'acide naphtalène sulfonique, d'acide phénolsulfonique et de formaldéhyde | 30 g |
| — kaolin | 100 g |
| — craie de champagne | 120 g |

Une autre composition de poudre à pulvériser à 40% utilise les constituants suivants:

| | |
|---|---|
| — matière active | 400 g |
| — lignosulfonate de sodium | 50 g |
| — dibutylnaphtalène sulfonate de sodium | 10 g |
| — silice | 540 g |

Une autre composition de poudre à pulvériser à 25% utilise les constituants suivants:

| | |
|---|---|
| — matière active | 250 g |
| — lignosulfonate de calcium | 45 g |
| — mélange équipondéral de craie de Champagne et d'hydroxyéthylcellulose | 19 g |
| — dibutylnaphtalène sulfonate de sodium | 15 g |
| — silice | 195 g |
| — craie de Champagne | 195 g |
| — kaolin | 281 g |

Une autre composition de poudre à pulvériser à 25% utilise les constituants suivants:

| | |
|---|---|
| — matière active | 250 g |
| — isooctylphénoxy-polyoxyéthylène-éthanol | 25 g |
| — mélange équipondéral de craie de Champagne et d'hydroxyéthylcellulose | 17 g |
| — aluminosilicate de sodium | 543 g |
| — kieselguhr | 165 g |

Une autre composition de poudre à pulvériser à 10% utilise les constituants suivants:

| | |
|---|---|
| — matière active | 100 g |
| — mélange de sels de sodium de sulfates d'acides gras saturés | 30 g |
| — produit de condensation d'acide naphtalène sulfonique et de formaldéhyde | 50 g |
| — kaolin | 820 g |

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses; on peut les mettre en suspension avec de l'eau à toute concentration désirée et cette suspension est utilisable très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une »mayonnaise«.

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25% de matière active et 0 à 10% d'additifs comme des stabilisants, des agents de modification à libération lente, des liants et des solvants.

Selon un exemple de composition de granulé, on utilise les constituants suivants:

| | |
|---|---|
| — matière active | 50 g |
| — épichlorhydrine | 2,5 g |
| — éther de cétyle et de polyglycol | 2,5 g |
| — polyéthylène glycol | 35 g |
| — kaolin (granulométrie: 0,3 à 0,8 mm) | 910 g |

Dans ce cas particulier on mélange la matière active avec l'épichlorhydrine et on dissout avec 60 g d'acétone; on ajoute alors le polyéthylène glycol et l'éther de cétyle et de polyglycol. On arrose le kaolin avec la solution obtenue et on évapore ensuite l'acétone sous vide. On utilise avantageusement un tel microgranulé pour lutter contre les champignons du sol.

Les composés de formule (I) peuvent encore être utilisés sous forme de poudres pour poudrage; on peut aussi utiliser une composition comprenant 50 g de matière active et 950 g de talc; on peut aussi utiliser une composition comprenant 20 g de matière active, 10 g de silice finement divisée et 970 g de talc; on mélange et broie ces constituants et on applique le mélange par poudrage.

**Revendications**

1. Produits caractérisés en ce qu'ils ont pour formule:

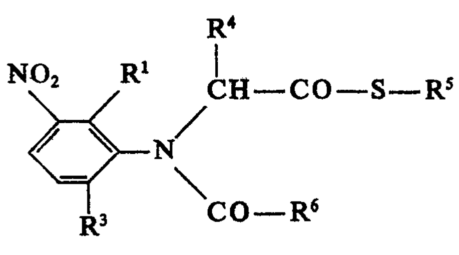

(I)

dans laquelle:

$R^1$ et $R^3$ identiques ou différents, représentent un radical alkyle ayant de 1 à 4 atomes de carbone et sont de préférence le radical méthyle,

$R^4$ représente l'atome d'hydrogène ou un radical méthyle,

$R^5$ représente l'atome d'hydrogène ou un radical alkyle ayant de 1 à 4 atomes de carbone,

$R^6$ représente un radical alkyle, éventuellement mono ou dihalogéné ayant de 1 à 6 atomes de carbone, ou un radical cycloalkyle ayant de 3 à 6 atomes de carbone ou un radical $-CH_2-X-R^7$,

X représente l'atome d'oxygène ou de soufre,
$R^7$ représente un radical alkyle ayant de 1 à 4 atomes de carbone.

2. Produits selon la revendication 1 caractérisés en ce que $R^1$, $R^3$, $R^4$ et, le cas échéant, $R^7$ sont le groupe méthyle.

3. Produits selon l'une des revendications 1 ou 2 caractérisés en ce que $R^6$ est le groupe $-CH_2-X-R^7$.

4. Produit selon l'une des revendications 1 à 3 caractérisé en ce qu'il est sous forme d'un stéréoisomère ou diastéréoisomère.

5. Procédé de préparation de composés de formule:

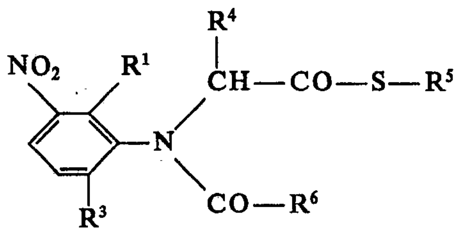

(I)

dans laquelle les divers symboles ont les significations données dans l'une des revendications 1 à 3 caractérisé en ce qu'on fait agir un chlorure d'acide de formule $R^6-CO-Cl$ (III) avec un composé de formule:

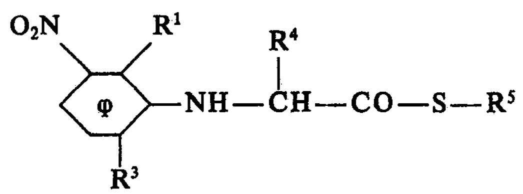

(II)

6. Procédé selon la revendication 5 caractérisé en ce qu'on opère en milieu solvant et à température comprise entre 40° C et la température de dégradation des réactifs et/ou produits de réaction, cette température étant de préférence comprise entre 60 et 150° C.

7. Procédé selon l'un des revendications 5 ou 6 caractérisé en ce qu'on opère en présence d'un catalyseur, de préférence le diméthylformamide ou un accepteur d'acide.

8. Procédé selon l'une des revendications 6 à 7 caractérisé en ce qu'on introduit le chlorure d'acide de formule (III) dans le composé de formule (II).

9. Procédé de préparation de composés de formule:

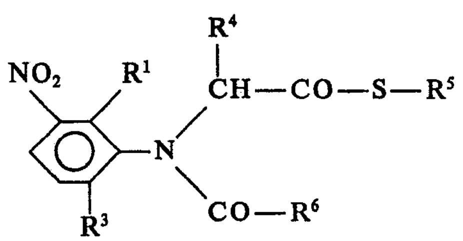

(I)

dans laquelle les divers symboles ont les significations données dans l'une des revendications 1 à 3 caractérisé en ce qu'on fait réagir un mercaptan de formule $R^5SH$ sur un acide de formule (IV)

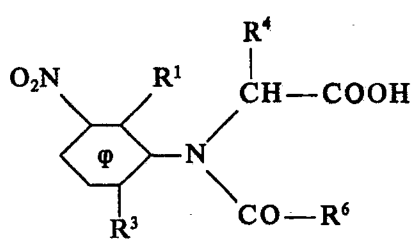

10. Procédé selon la revendication 9 caractérisé en ce que la réaction s'effectue entre 10 et 120° C dans un solvant organique inerte.

11. Procédé selon l'une des revendications 9 ou 10 caractérisé en ce qu'on opère en présence d'un agent de déshydratation, de préférence un carbodiimide.

12. Compositions, utilisables pour la protection des végétaux contre les maladies fongiques, caractérisées en ce qu'elles contiennent, comme matière active, un composé selon l'une des revendications 1 à 4 en association avec les supports inertes et usuels et éventuellement les agents tensio-actifs usuels.

13. Compositions selon la revendications 12, caractérisées en ce qu'elles contiennent $5 \cdot 10^{-5}$ à 95% (en poids) de matière active.

14. Procédé de lutte contre les maladies fongiques des plantes caractérisé en ce que l'on utilise une composition selon l'une des revendications 12 ou 13.

**Patentansprüche**

1. Produkte, dadurch gekennzeichnet, daß sie der Formel

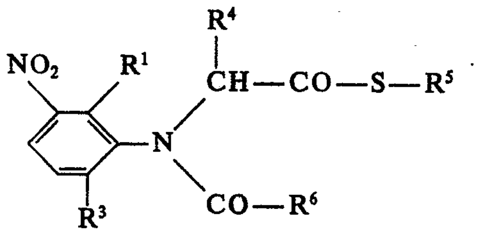

(I)

entsprechen, in der

| | |
|---|---|
| $R^1$ und $R^3$ | die gleich oder verschieden sind, eine Alkylgruppe mit 1 bis 4 Kohlenstoffatomen, vorzugsweise die Methylgruppe bedeuten, |
| $R^4$ | ein Wasserstoffatom oder eine Methylgruppe bedeutet, |
| $R^5$ | ein Wasserstoffatom oder eine Alkylgruppe mit 1 bis 4 Kohlenstoffatomen steht, |
| $R^6$ | eine Alkylgruppe mit 1 bis 6 Kohlenstoffatomen, die gegebenenfalls durch 1 oder 2 Halogenatome substituiert ist, oder eine Cycloalkylgruppe mit 3 bis 6 Kohlenstoffatomen oder eine Gruppe $-CH_2-X-R^7$ ist, |
| X | für ein Sauerstoff- oder Schwefelatom steht und |
| $R^7$ | eine Alkylgruppe mit 1 bis 4 Kohlenstoffatomen bedeutet. |

2. Produkte nach Anspruch 1, dadurch gekennzeichnet, daß $R^1$, $R^3$, $R^4$ und gegebenenfalls $R^7$ jeweils die Methylgruppe bedeuten.

3. Produkte nach einem der Ansprüche 1 oder 2, dadurch gekennzeichnet, daß $R^6$ die Gruppe $-CH_2-X-R^7$ ist.

4. Produkt nach einem der Ansprüche 1 bis 3, dadurch gekennzeichnet, daß es in Form eines Stereoisomeren oder Diastereoisomeren vorliegt.

5. Verfahren zur Herstellung von Verbindungen der Formel

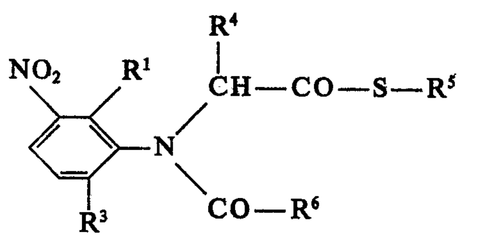

(I)

in der die verschiedenen Symbole die in einem der Ansprüche 1 bis 3 angegebenen Bedeutungen haben, dadurch gekennzeichnet, daß man ein Säurechlorid der Formel $R^6CO-Cl$ (III) mit einer Verbindung der Formel

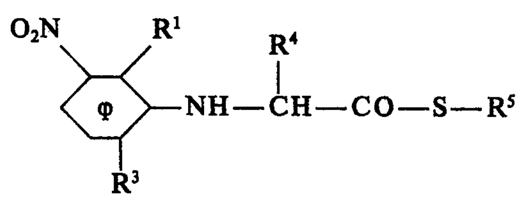

(II)

zur Umsetzung bringt.

6. Verfahren nach Anspruch 5, dadurch gekennzeichnet, daß man in einem Lösungsmittelmedium arbeitet sowie bei einer Temperatur zwischen 40° C und der Zersetzungstemperatur der Reaktionspartner und/oder Reaktionsprodukte, wobei diese Temperatur vorzugsweise im Bereich von 60 bis 150°C liegt.

7. Verfahren nach einem der Ansprüche 5 oder 6, dadurch gekennzeichnet, daß man in Gegenwart eines Katalysators, vorzugsweise in Gegenwart von Dimethylformamid oder eines Säureakzeptors arbeitet.

8. Verfahren nach einem der Ansprüche 6 bis 7, dadurch gekennzeichnet, daß man das Säurechlorid der Formel (III) in die Verbindung der Formel (II) einbringt.

9. Verfahren zur Herstellung von Verbindungen der Formel

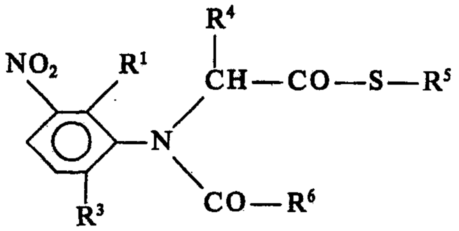

(I)

bei denen die verschiedenen Symbole die in einem der Ansprüche 1 bis 3 angegebenen Bedeutungen haben, dadurch gekennzeichnet, daß man ein Mercaptan der Formel $R^5SH$ mit einer Säure der Formel

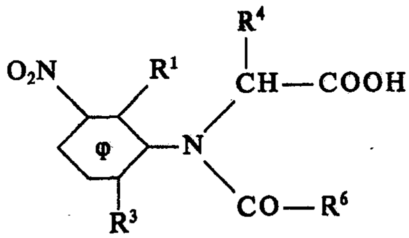

(IV)

umsetzt.

10. Verfahren nach Anspruch 9, dadurch gekennzeichnet, daß die Reaktion zwischen 10 und 120°C in einem inerten organischen Lösungsmittel ausgeführt wird.

11. Verfahren nach einem der Ansprüche 9 oder 10, dadurch gekennzeichnet, daß man in Gegenwart eines Dehydratationsmittels, vorzugsweise eines Carbodiimids, arbeitet.

12. Mittel, verwendbar für den Schutz von Pflanzen gegen Pilzkrankheiten, dadurch gekennzeichnet, daß sie als Wirkstoff eine Verbindung nach einem der Ansprüche 1 bis 4, kombiniert mit den gebräuchlichen inerten Trägern und gegebenenfalls den gebräuchlichen grenzflächenaktiven Mitteln, enthalten.

13. Mittel nach Anspruch 12, dadurch gekennzeichnet, daß sie $5 \cdot 10^{-5}$ bis 95 Gew.-% Wirkstoff enthalten.

14. Verfahren zum Bekämpfen der Pilzkrankheiten von Pflanzen, dadurch gekennzeichnet, daß man ein Mittel nach einem der Ansprüche 12 oder 13 verwendet.

**Claims**

1. Compounds characterized in that they have the formula:

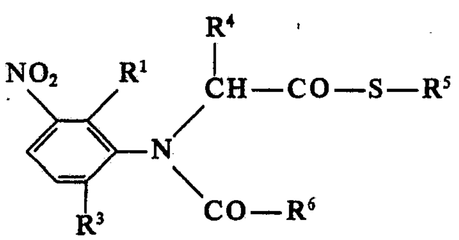

(I)

in which:

- $R^1$ and $R^3$ which are identical or different, represent an alkyl radical having from 1 to 4 carbon atoms and are preferably the methyl radical,
- $R^4$ represents the hydrogen atom or a methyl radical,
- $R^5$ represents the hydrogen atom or an alkyl radical having from 1 to 4 carbon atoms,

— $R^6$ represents an optionally monohalogenated or dihalogenated alkyl radical having from 1 to 6 carbon atoms, a cycloalkyl radical having from 3 to 6 carbon atoms or a radical $-CH_2-X-R^7$,

— X represents the oxygen or sulphur atom, and

— $R^7$ represents an alkyl radical having from 1 to 4 carbon atoms.

2. Compounds according to claim 1, characterized in that $R^1$, $R^3$, $R^4$ and, if appropriate, $R^7$ are the methyl group.

3. Compounds according to one of claims 1 or 2, characterized in that $R^6$ is the group $-CH_2-X-R^7$.

4. Compounds according to one of claims 1 to 3, characterized in that it is in the form of a stereoisomer or diastereoisomer.

5. A process for the preparation of compounds of the formula:

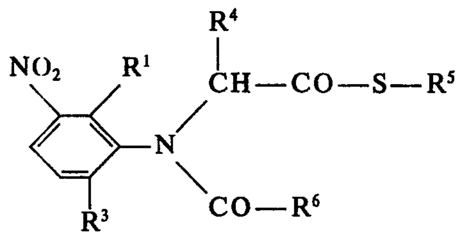

(I)

in which the various symbols have the meanings given in one of claims 1 to 3, characterized in that an acid chloride of the formula $R^6-CO-Cl$ (III) is reacted with a compound of the formula:

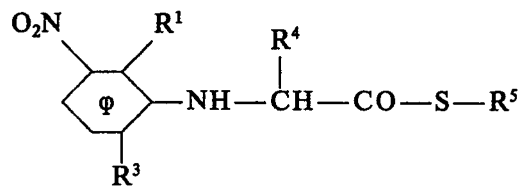

(II)

6. A process according to claim 5, characterized in that the reaction is carried out in a solvent medium and at a temperature of between 40°C and the degradation temperature of the reactants and/or reaction products, this temperature preferably being between 60 and 150°C.

7. A process according to one of claims 5 or 6, characterized in that the reaction is carried out in the presence of a catalyst, preferably dimethylformamide or an acid acceptor.

8. A process according to one of claims 6 and 7, characterized in that the acid chloride of the formula (III) is introduced into the compound of the formula (II).

9. A process for the preparation of compounds of the formula:

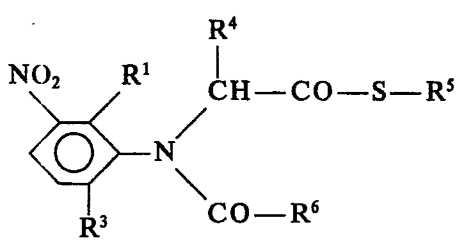

(I)

in which the various symbols have the meanings given in one of claims 1 to 3, characterized in that a mercaptan of the formula $R^5SH$ is reacted with an acid of the formula (IV):

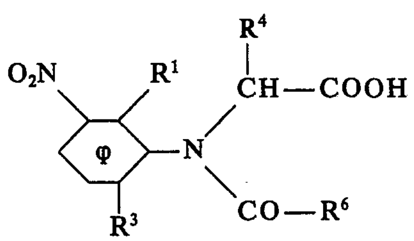

10. A proces according to claim 9, characterized in that the reaction is carried out at between 10 and 120°C in an inert organic solvent.

11. A process according to one of claims 9 or 10, characterized in that the reaction is carried out in the presence of a dehydrating agent, preferably a carbodiimide.

12. Compositions which can be used for protecting plants against fungal diseases, characterized in

that they contain, as the active ingredient, a compound according to one of claims 1 to 4, in association with the customary inert carriers and, optionally, the customary surface-active agents.

13. Compositions according to claim 12, characterized in that they contain $5 \cdot 10^{-5}$ to 95% by weight for active ingredient.

14. A process for combating fungal diseases of plants, characterized in that a composition according to one of claims 12 or 13 is used.